# EUROPÄISCHE PATENTSCHRIFT

(11) **EP 2 954 785 B1**
(45) Veröffentlichungstag und Bekanntmachung des Hinweises auf die Patenterteilung: **06.06.2018**
(21) Anmeldenummer: 14172306.4
(22) Anmeldetag: 13.06.2014
(51) Int. Cl.: A23L 27/00, A23L 27/30, C07H 15/24, C12P 19/18, C12P 19/56

(54) **Neue Stoffmischung zur Verbesserung des Süssgeschmacks enthaltend Rubusosid oder alpha-Glycosylrubusosid**
New composition for improvement of sweet taste comprising rubusoside or alpha-glycolsylrubusoside
Composition nouveau pour amélioration de gout doux contenant de rubusoside or alpha-glycosylrubusoside

(43) Veröffentlichungstag der Anmeldung: 16.12.2015
(73) Patentinhaber: Symrise AG, 37603 Holzminden (DE)
(72) Erfinder: Ley, Jakob, 37603 Holzminden (DE); Geißler, Torsten, 37575 Einbeck (DE); Krammer, Gerhard, 37603 Holzminden (DE); Sabater, Christopher, 37603 Holzminden (DE); Riess, Thomas, 37603 Holzminden (DE)
(74) Vertreter: Eisenführ Speiser

(56) Entgegenhaltungen:
- EP-A1- 2 353 403
- EP-A1- 2 386 211
- WO-A1-2012/112180
- JP-A- 2002 045 145
- MUCHSIN DARISE ET AL: "Enzymic Transglucosylation of Rubusoside and the Structure-Sweetness Relationship of Steviol-Bisglycosides", AGRICULTURAL AND BIOLOGICAL CHEMISTRY., Bd. 48, Nr. 10, 1. Januar 1984 (1984-01-01), Seiten 2483-2488, XP55131924, TOKYO, JP ISSN: 0002-1369, DOI: 10.1271/bbb1961.48.2483
- KAZUHIRO OHTANI ET AL: "Further study on the 1,4-.ALPHA.-transglucosylation of rubusoside, a sweet steviol-bisglucoside from Rubus suavissimus.", AGRICULTURAL AND BIOLOGICAL CHEMISTRY, Bd. 55, Nr. 2, 1. Februar 1991 (1991-02-01), Seiten 449-453, XP055137190, ISSN: 0002-1369, DOI: 10.1271/bbb1961.55.449
- GUIXIN CHOU ET AL: "Quantitative and Fingerprint Analyses of Chinese Sweet Tea Plant (Rubus suavissimus S. Lee)", JOURNAL OF AGRICULTURAL AND FOOD CHEMISTRY, Bd. 57, Nr. 3, 11. Februar 2009 (2009-02-11), Seiten 1076-1083, XP055004462, ISSN: 0021-8561, DOI: 10.1021/jf8029397

## Beschreibung

### GEBIET DER ERFINDUNG

Die Erfindung befindet sich auf dem Gebiet der Nahrungsmittel und betrifft neue Stoffmischungen, die als Aromazubereitungen zur Verbesserung des Süßgeschmacks in oral konsumierbaren Zubereitungen eingesetzt werden.

### STAND DER TECHNIK

Rubusglykoside werden aus den Blättern der Pflanze *Rubus suavissimus S. Lee* (chinesische Brombeere) gewonnen und stellt ein komplexes Gemisch verschiedener Glykoside des Diterpens Steviol dar. Rubusglykoside werden als kalorienfreie Süßungsmittel eingesetzt, wobei reines Rubusosid die 200fache Süßkraft von Zucker aufweist. Die chinesische Brombeere ist in Südostasien beheimatet, wo ihre Süßkraft schon seit langem bekannt ist. Heutzutage wird die Pflanze in großem Maßstab in den chinesischen Regionen Guang-xi und Guangdong angebaut.

Aus dem Stand der Technik sind Produkte, die Rubusglykoside als Süßungsmittel, so genannte "Sweetener" enthalten, hinreichend bekannt. Ebenfalls bekannt sind Unternehmungen, den Gehalt von Rubusosid durch Anreicherungsverfahren zu erhöhen. So werden beispielsweise in den beiden Patentanmeldungen EP 2641479 und WO 2012177727 Mischungen von Rubusosid und Steviosiden sowie deren Verwendung als Sweetener beschrieben.

Gegenstand der WO 2013 133689 A1 ist ein chromatographisches Verfahren zur Aufreinigung von Steviolglykosiden.

Aus der JP 2002 045145 A (TOYO) ist die Herstellung von Rubusosid-Glucosiden bekannt sowie deren Einsatz in Aromamischungen.

WO 2012 112180 A1 (PURECIRCLE) beschreibt glucosylierte Steviaderivate, die auch Rubusoside enthalten können. Entsprechende Stoffe und deren Herstellung mit Hilfe einer CGTase sind auch aus dem Aufsatz von MUCHSIN DARISE ET AL in AGRICULTURAL AND BIOL. CHEM. 48(10), S 2483-2488 (1984**)** bekannt.

Dennoch zeichnen sich im Markt befindlichen Qualitäten für Rubusglykoside dadurch aus, dass sie insgesamt einen bitteren, zuweilen adstringierenden Nachgeschmack sowie eine unzureichende Süßungswirkung aufweisen, was ihren breiten Einsatz nach wie vor erschwert. Rubusglycoside können zur Maskierung unangenehmer Geschmackseindrücke verwendet werden (EP 2,386,211), allerdings funktioniert das nicht mit sich selbst. Zubereitungen zur Verbesserung mit einfachen Rubus-Extrakten wurden beschrieben (EP 2,641,479), führen aber nicht zur Verbesserung des Süßprofils.

Zwar wurden schon früher einzelne Produkte einer alpha-Glycosilierung von Rubusosid isoliert und geschmacklich bewertet (KAZUHIRO OHTANI ET AL in AGRICULTURAL AND BIOL. CHEM 55(2), S. 449-453 (1991**)),** allerdings ist die Aufreinigung bis zum Einzelstoff extrem aufwändig und kommerziell nicht sinnvoll und somit eine Anwendbarkeit nicht gegeben.

Die Aufgabe der vorliegenden Erfindung hat somit darin bestanden, neue Stoffmischungen bereit zu stellen, die den Süßgeschmack von oralen Zubereitungen verstärken, verbessern oder abrunden. Insbesondere bestand eine Aufgabe der vorliegenden Erfindung darin, zum einen das geschmacklich sensorische Profil von Rubusglykoside durch einen natürlichen (enzymatischen, fermentativen oder küchentechnischen) Prozess abzurunden, wobei neue Aromazubereitungen erhalten werden sollten. Zum Anderem bestand eine weitere Aufgabe der Erfindung darin, alpha-Glucosylrubusoside durch natürliche Prozesse zugänglich zu machen. Die so gewonnenen alpha-Glucosylrubusoside zeichnen sich neben der Abrundung des geschmacklichen Profils durch ihre Wirksamkeit in geringen Konzentrationen, sowie durch die Eigenschaft der Verstärkung des Süßgeschmacks anderer Stoffe aus.

### BESCHREIBUNG DER ERFINDUNG

Gegenstand der Erfindung ist daher eine Stoffmischung, Stoffmischung, enthaltend mindestens eine Verbindung der allgemeinen Formel (I), 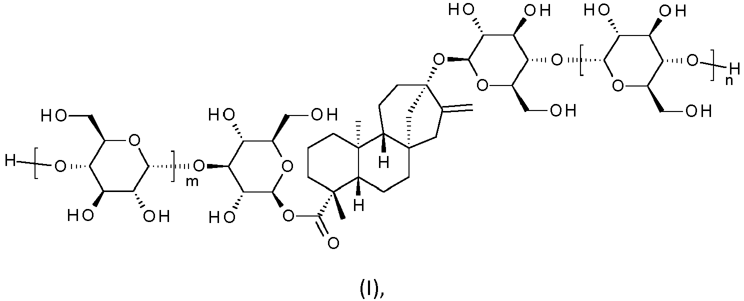 wobei
m und n unabhängig voneinander 0 bis 50 darstellen,
und wobei im Falle von m = n = 0 mindestens eine weitere Verbindung der allgemeinen Formel (I) mit m oder n ≥ 1 anwesend ist, die sich dadurch auszeichnet, dass sie
   (a1) weniger als 50 Gew.-% Rubusosid (m + n = 0),
   (a2) mehr als 10 Gew.-% alpha-Glucosylrubusoside der oben angegebenen allgemeinen Formel (I) mit insgesamt 3 Glucoseeinheiten (m + n = 1),
   (a3) mehr als 15 Gew.-% alpha-Glucosylrubusoside der allgemeinen Formel (I) mit insgesamt 4 Glucoseeinheiten (m + n = 2),
   (a4) mehr als 5 Gew.-% alpha-Glucosylrubusoside der allgemeinen Formel (I) mit insgesamt 5 Glucoseeinheiten (m + n = 3), und
   (a5) weniger als 6 Gew.-% Steviolmonosid,
   aufweist,
   wobei die Menge aller Komponenten (a1) bis (a5) sich zusammen zu mindestens 80 Gew.-% addiert und die restliche Menge sich aus Wasser, Glycerin, Stärkeabbauprodukten, Proteinen, Fettsäuren und/ oder Fettsäureestern zusammensetzt.

### RUBUSGLYKOSIDE

Rubusglykoside können aus der Pflanze *Rubus suavissimus S. Lee* (chinesische Brombeere) zum Beispiel EP 223 6043 gewonnen werden und bestehen zumindest aus dem Diterpenglycosid Rubusosid, bevorzugt mit einem Anteil von 1 bis 100 Gew.-%, besonders bevorzugt 10 bis 95 Gew.-%, besonders bevorzugt 30 bis 75 Gew.-% jeweils bezogen auf die Gesamtmenge an Rubusglykosiden und kann eins oder mehrere der bisher 13 bekannten weiteren Isomeren und Homologen, wie Suaviosid A, B und R1 enthalten, die aber teilweise auch bitter schmecken (Hirono, S.; Chou, W. H.; Kasai, R.; Tanaka, O.; Tada, T., Sweet and bitter diterpene glucosides from leaves of Rubus suavissimus. Chemical & Pharmaceutical Bulletin 1990, 38, (6), 1743-1744 und Chou, G.; Xu, S.-J.; Liu, D.; Koh, G. Y.; Zhang, J.; Liu, Z., Quantitative and Fingerprint Analyses of Chinese Sweet Tea Plant (Rubus suavissimus S. Lee). J. Agric Food Chem. 2009, 57, 1076-1083.).

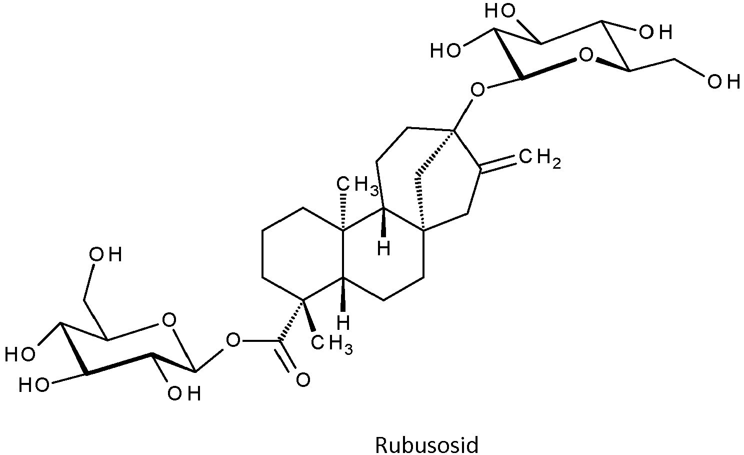

Die Anteile der enthaltenen Rubusglykoside unterscheiden sich nach Anbaugebiet und Pflanzensorte. Dem Lakritz artigen Geschmack der Pflanze wird bei der Herstellung des Süßstoffgemisches durch Isolierung der süßenden Bestandteile und anschließender Komposition entgegengewirkt. Rubusprodukte haben normalerweise einen Anteil 10% - 70 % Rubusosid. Rubusosid selbst kann auch durch enzymatische Konversion aus Steviosiden gewonnen werden, wie in WO 2013 133689 beschrieben.

In der vorliegenden Erfindung wird im Gegensatz zum Stand der Technik bei der Herstellung von Rubusglykosiden in der Transglucosylierungs-Reaktion mit einem 1,4-glykosidischen Kohlenhydrat, welche vorzugsweise Stärke ist, bevorzugt das Enzym alpha-Cyclodextrin glucanotransferase (CGTase) eingesetzt. So ist es möglich Stevia-Extrakte zu modifizieren, so dass alpha-Glycosylstevioside entstehen, wie z.B. in WO 2012 112180 beschrieben. Zur Erzeugung von Rubus-Extrakten mit verbessertem Geschmack in Sinne dieser Erfindung wurde diese Reaktion noch nicht eingesetzt. Demgemäß ist ein weiterer Gegenstand der vorliegenden Erfindung ein Verfahren zur Herstellung von Rubusglykoside, welche einer erfindungsgemäße Stoffmischung enthalten sind, umfassend die Verwendung von alpha-Cyclodextrin glucanotransferase in der Transglucosylierungs-Reaktion mit einem 1,4-glykosidischen Kohlenhydrat, welche vorzugsweise Kohlenhydrat ist.

### ALPHA-GLUCOSYLRUBUSOSIDE

alpha-Glucosylrubusoside sind enzymatisch, fermentativ oder chemisch modifizierte Rubusglykoside, insbesondere Rubusoside der allgemeinen Formel (I) und weisen neben den im Rubusosid schon enthaltenden beiden Glucose-Einheiten (m = n = 0) mindestens 1 bis 20, vorzugsweise mindestens 2 bis 10 und insbesondere 2 bis 6 zusätzliche Glucoseeinheiten auf, wobei diese zusätzlichen Glucoseeinheiten bevorzugt als alpha-1,4-verknüpfte D-Glucopyranoseeinheiten vorliegen.

Überraschenderweise wurde gefunden, dass die erfindungsgemäßen Stoffmischungen der Verbindung mit der allgemeinen Formel (I) im Vergleich zu Rubusglykosiden alleine nicht nur über eine wesentlich verbesserte Süßungswirkung verfügt, sondern ausgesprochen gut geeignet ist, die Anfangssüße und das Mundgefühl von oral konsumierbaren Zubereitungen zu verbessern, verstärken und zu optimieren. Ebenfalls zeigte sich ein verbessertes Geschmacksprofil, insbesondere im Hinblick auf die Süßungswirkung im Vergleich von Zubereitungen, die die erfindungsgemäße Stoffmischung enthalten und Zubereitungen mit reduziertem Anteil an Saccharose oder die anderer süß schmeckender Kohlenhydrate enthalten. Insbesondere zeigte es sich überraschenderweise, dass die erfindungsgemäße Stoffmischung in der Kombination mit phenolischen süßverstärkenden Aromastoffen und auch Stärkeabbauprodukten, der teilweise unangenehme süßstoffartige Nachgeschmack der Rubusglykoside und/oder alpha-Glucosylrubusoside reduziert und/oder überdeckt werden konnte.

In einer weiteren bevorzugten Ausführungsform der vorliegenden Erfindung umfasst ein erfindungsgemäßes Stoffgemisch, neben mindestens eine Verbindung der Formel (I) wobei m und n unabhängig voneinander 0 bis 50 darstellen, und wobei im Falle von m = n = 0 mindestens eine weitere Verbindung der allgemeinen Formel (I) mit m oder n ≥ 1 anwesend ist, zusätzliche Stärkeabbauprodukte. Stärkeabbauprodukte sind hierbei vorzugsweise Stärkeabbauprodukte, die 3 bis 100 jeweils alpha-1-4-verknüpften D-Glucopyranosylresten enthalten.

### STÄRKEABBAUPRODUKTE

Geeignete Stärkeabbauprodukte haben in den erfindungsgemäßen Mischungen die Funktion von Trägerstoffen. Vorzugsweise handelt es sich dabei um Dextrine bzw. Maltodextrine, die von ihrer Molekülgröße her zwischen Oligosacchariden und Stärke liegen. Üblicherweise kommen sie in Form von weißem bzw. hellgelbem Pulver vor. Sie werden hauptsächlich aus Weizen-, Kartoffel- und Maisstärke durch trockene Erhitzung (>150 °C) oder unter Säureeinwirkung gewonnen. In der Natur wird Dextrin zum Beispiel von *Bacterium macerans* erzeugt. Dextrine entstehen auch durch den enzymatischen Abbau von Stärke durch Amylase.

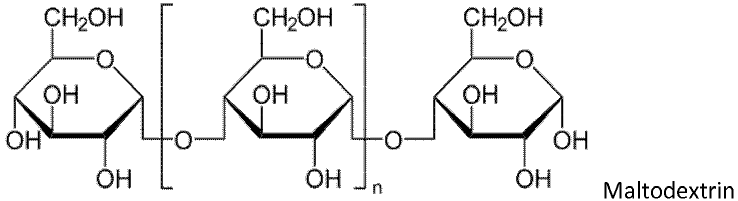

Maltodextrine, die in besonderer Weise geeignet sind, weisen etwa 3 bis etwa 20, vorzugsweise etwa 5 bis etwa 15 Dextrose-Äquivalente (DE) auf. Hierunter ist der prozentuale Anteil der reduzierenden Zucker in der Trockenmasse zu verstehen.

In einer weiteren bevorzugten Ausführungsform der vorliegenden Erfindung umfasst ein erfindungsgemäßes Stoffgemisch zusätzlich ein oder mehrere phenolische, natürlich vorkommende süßverstärkende Aromastoffe umfasst, ausgewählt aus der Gruppe, die gebildet wird von Hesperetin, Phloretin, 1-(2,4-Dihydroxy-phenyl)-3-(3-hydroxy-4-methoxy-phenyl)-propan-1-on, 7,3-Dihydroxy-4'methoxyflavan und 5-Hydroxy-4-(4-hydroxy-3-methoxyphenyl)-7-methoxy-2-chromanon.

Im erfindungsgemäßen Stoffgemisch stellen die Verbindungen der Formel (I) vorzugsweise Rubusglykoside dar, welche bevorzugt Rubososid (m = n = 0) ist oder besonders bevorzugt eines der alpha-Glycosylrubusoside der Formel (II) ist 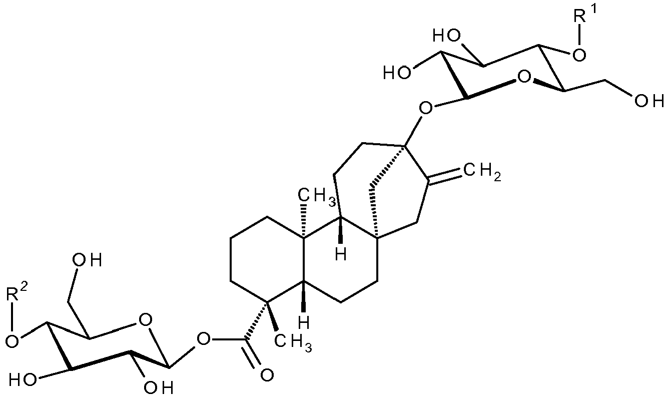 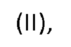 wobei die Reste R1 und R2 die folgenden Reste darstellen:

| **Verbindung** | **Rubusglykosid** | **R¹** | **R²** |
|---|---|---|---|
| 1 | Rubustriglucosid | H- | Glcα1- |
| 2 | Rubustriglucosid | Glcα1- | H- |
| 3 | Rubustetraglucosid | Glcα1- | Glcα1- |
| 4 | Rubustetraglucosid | -Glcα1-4Glcα | H- |
| 5 | Rubustetraglucosid | H- | -Glcα1-4Glcα |
| 6 | Rubuspentaglucosid | -Glcα1-4Glcα | Glcα1- |
| 7 | Rubuspentaglucosid | Glcα1- | -Glcα1-4Glcα |
| 8 | Rubushexaglucosid | -Glcα1-4Glcα | -Glcα1-4Glcα |
| 9 | Rubuspentaglucosid | -Glcα1-4Glcα1-4Glcα | H- |
| 10 | Rubuspentaglucosid | H- | -Glcα1-4Glcα1-4Glcα |
| 11 | Rubushexaglucosid | -Glcα1-4Glcα1-4Glcα | Glcα1- |
| 12 | Rubushexaglucosid | Glcα1- | -Glcα1-4Glcα1-4Glcα |
| 13 | Rubusheptaglucosid | -Glcα1-4Glcα | -Glcα1-4Glcα1-4Glcα |
| 14 | Rubusheptaglucosid | -Glcα1-4Glcα1-4Glcα | -Glcα1-4Glcα |
| 15 | Rubusoctaglucosid | -Glcα1-4Glcα1-4Glcα | -Glcα1-4Glcα1-4Glcα |

Selbstverständlich sind Mischungen der Verbindungen von 1 bis 15 und / oder Rubusosid ebenfalls möglich und erfindungsgemäß. Demgemäß ist eine bevorzugte Ausführungsform ein Stoffgemisch mit mindestens eine Verbindung der Formel (I), welche ausgewählt ist aus einen der Verbindungen der Formel (II):
- Rubustriglucosid (Verbindung 1),
- Rubustriglucosid (Verbindung 2),
- Rubustetraglucosid (Verbindung 3),
- Rubustetraglucosid (Verbindung 4),
- Rubustetraglucosid (Verbindung 5),
- Rubuspentaglucosid (Verbindung 6),
- Rubuspentaglucosid (Verbindung 7),
- Rubushexaglucosid (Verbindung 8),
- Rubuspentaglucosid (Verbindung 9),
- Rubuspentaglucosid (Verbindung 10),
- Rubushexaglucosid (Verbindung 11),
- Rubushexaglucosid (Verbindung 12),
- Rubusheptaglucosid (Verbindung 13),
- Rubusheptaglucosid (Verbindung 14),
- Rubusoctaglucosid (Verbindung 15).

Vorzugsweise enthalten die erfindungsgemäßen Stoffmischungen alpha-Glucosy-Irubusoside der Formel I, die die folgende Zusammensetzung aufweisen:
(a1) weniger als 30 Gew.-% Rubusosid (m + n = 0),
(a2) mehr als 15 Gew.-% alpha-Glucosylrubusoside mit insgesamt 3 Glucoseeinheiten (m + n = 1),
(a3) mehr als 18 Gew.-% alpha-Glucosylrubusoside mit insgesamt 4 Glucoseeinheiten (m + n = 2).
(a4) mehr als 10 Gew.-% alpha-Glucosylrubusoside mit insgesamt 5 Glucoseeinheiten (m + n = 3),
(a5) weniger als 2,5 Gew.-% Steviolmonosid,
wobei die Menge aller Komponenten (a1) bis (a5) sich zusammen zu mindestens 80 Gew.-%, bevorzugt mindestens 90 Gew.-% addiert. Die restliche Menge von 10 bis 20 Gew.-% setzt sich hierbei generell aus Wasser, Glycerin, Stärkeabbauprodukten, Proteinen, Fettsäuren und/ oder Fettsäureestern zusammen.

Vorzugsweise weisen die erfindungsgemäßen Stoffgemische zwischen der Komponente(n) der Verbindung(en) der Formel (I) und der zusätzlichen Stärkeabbauprodukte ein Gewichtsverhältnis von 1:99 bis 99:1, vorzugsweise von 5:95 bis 75:25, besonders bevorzugt von 25:75 bis 75:25 und ganz besonders bevorzugt von 5:95 bis 25:75, auf.

### HERSTELLUNG

Die erfindungsgemäße Stoffgemische, umfassend Verbindungen der Formel I (alpha-Glucosylrubusoside) sind prinzipiell bekannt und lassen sich zum Beispiel gemäß Ohtani, K.; Aikawa, Y.; Ishikawa, H.; Kasai, R.; Kitahata, S.; Mizutani, K.; Doi, S.; Nakaura, M.; Tanaka, O., Further study on the 1,4-alpha-transglucosylation of rubusoside, a sweet steviol-bisglucoside from Rubus suavissimus. Agric. Biol. Chem. 1991, 55, (2), 449-453 herstellen, indem man
(i) Rubusglycoside mit einem alpha-Glucan, bevorzugt Stärke, einem Stärkeabbauprodukt oder Cyclodextrinen mit einer Glycosidhydrolase, bevorzugt einer alpha-Glycosidase wie beispielsweise alpha-Amylase, alpha-Glucosidase, beta-Amylase, Cyclomaltodextrin glucanotransferase oder einem geeigneten Mikroorganismus, der solche Enzyme aktiv homolog oder heterolog exprimiert (z.B. *Xantomonas campestris, Saccharomyces cerevisiae* oder *Leuconostoc mensenteroides*) bei 0°C bis 90°C, bevorzugt 5°C bis 80°C, besonders bevorzugt 20°C bis 65°C, bevorzugt in einem Wasser enthaltenden Medium für 0 bis 48 Stunden, bevorzugt 0,1 bis 24 Sunden, besonders bevorzugt 0,5-16 Stunden umsetzt und
(ii) das erhaltene Gemisch deaktiviert, pasteurisiert oder sterilisiert, indem man es auf 70°C bis 150 °C bei normalem oder erhöhtem Umgebungsdruck für 5 bis 500 min erhitzt und
(iii) die erhaltene Mischung dann trocknet und dabei z.B. Sprühtrocknung, Gefriertrocknung, destillative Trocknung, Bandtrocknung verwendet und
(iv) optional das Gemisch weiter aufreinigt und dabei z.B. Chromatographie, Verteilungsverfahren, Membranverfahren oder Kristallisation verwendet.

### ORALE ZUBEREITUNGEN

Ein weiterer Gegenstand der vorliegenden Erfindung betrifft Zubereitungen zur oralen Aufnahme, die die erfindungsgemäße(n) Stoffmischung(en) in einer Menge von 0,00001 Gew.-% bis etwa 2 Gew.-% enthalten. Bevorzugt werden die erfindungsgemäße(n) Stoffmischung(en) in einer Menge von 0,0001 Gew.-% bis 1,5 Gew.-%, insbesondere bevorzugt von 0,001 Gew.-% bis 1 Gew.-%, ganz besonders bevorzugt von 0,01 Gew.-% bis 0,5 Gew.-% und insbesondere ganz besonders bevorzugt von 0,05 Gew.-% bis 0,1 Gew.-%

### NAHRUNGSMITTEL

Die oralen Zubereitungen, in denen die erfindungsgemäßen Stoffmischungen eingesetzt werden, können ausgewählt sein aus der Gruppe, die gebildet wird von
- Backwaren, beispielsweise Brot, Trockenkekse, Kuchen, sonstiges Gebäck,
- Süßwaren (beispielsweise Schokoladen, Schokoladenriegelprodukte, sonstige Riegelprodukte, Fruchtgummi, Hart- und Weichkaramellen, Kaugummi),
- alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke, (karbonisierte) Erfrischungsgetränke, Nektare, Schorlen, Obst- und Gemüsesäfte,
- Frucht- oder Gemüsesaftzubereitungen,
- Instantgetränke (beispielsweise Instant-Kakao-Getränke, Instant-Tee-Getränke, Instant-Kaffeegetränke, Instant-Fruchtgetränke),
- Fleischprodukte (beispielsweise Schinken, Frischwurst- oder Rohwurstzubereitungen, gewürzte oder marinierte Frisch- oder Pökelfleischprodukte),
- Eier oder Eiprodukte (Trockenei, Eiweiß, Eigelb), Getreideprodukte (beispielsweise Frühstückscerealien, Müsliriegel, vorgegarte Fertigreis-Produkte),
- Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte),
- Produkte aus Sojaprotein oder anderen Sojabohnen-Fraktionen (beispielsweise Sojamilch und daraus gefertigte Produkte, Fruchtgetränke mit Sojaprotein, Sojalecithinhaltige Zubereitungen, fermentierte Produkte wie Tofu oder Tempe oder daraus gefertigte Produkte),
- Produkte aus anderen pflanzlichen Proteinquellen, beispielsweise Haferprotein-Getränke,
- Fruchtzubereitungen (beispielsweise Konfitüren, Fruchteis, Fruchtsoßen, Fruchtfüllungen),
- Gemüsezubereitungen (beispielsweise Ketchup, Soßen, Trockengemüse, Tiefkühlgemüse, vorgegarte Gemüse, eingekochte Gemüse),
- Knabberartikel (beispielsweise gebackene oder frittierte Kartoffelchips oder Kartoffelteigprodukte, Extrudate auf Mais- oder Erdnussbasis),
- Produkte auf Fett- und Ölbasis oder Emulsionen derselben (beispielsweise Mayonnaise, Remoulade, Dressings),
- sonstige Fertiggerichte und Suppen (beispielsweise Trockensuppen, Instant-Suppen, vorgegarte Suppen),
- Gewürze, Würzmischungen sowie insbesondere Aufstreuwürzungen (englisch: Seasonings), die beispielsweise im Snackbereich Anwendung finden.

Besonders bevorzugt sind hier Süßwaren, Milchprodukte und ganz besonders bevorzugt sind nicht-alkoholische Getränke, wobei gesüßte Getränke besonders bevorzugt sind.

### HILFS- UND ZUSATZSTOFFE

Die oben genannten oralen Zubereitungen und Nahrungsmitteln können typischerweise weitere Hilfs- und Zusatzstoffe enthalten, darunter insbesondere Süßstoffe, Lebensmittelsäuren, Säureregulatoren, Verdickungsmittel und insbesondere weitere Aromastoffe.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
- synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-Tryptophan, L-Prolin);
- Weitere süß schmeckende niedermolekulare Substanzen, wie z.B. Hernandulcin, Dihydrochalconglykoside, insbesondere Neohesperidindihydrochalkon und Naringinchalkon, Glycyrrhizin, Glycerrhetinsäure, ihre Derivate und Salze, Extrakte von Lakritz (Glycyrrhizza glabra ssp.), *Lippia dulcis* Extrakte, *Momordica* ssp. Extrakte oder
- Einzelsubstanzen wie z.B. Momordica grosvenori [Luo Han Guo] und die daraus gewonnenen Mogroside, *Hydrangea dulcis* oder Extrakte Phyllodulcin oder
- Balansine aus *Mycetia balansae wie in* WO 2012 164,062 *beschrieben.*

### Lebensmittelsäuren

Geeignete Lebensmittelsäuren sind Carbonsäuren. Säuren im Sinne der Erfindung sind bevorzugt in Lebensmitteln zulässige Säuren, insbesondere die hier genannten:

| | |
|---|---|
| E 260 | - Essigsäure |
| E 270 | - Milchsäure |
| E 290 | - Kohlendioxid |
| E 296 | - Apfelsäure |
| E 297 | - Fumarsäure |
| E 330 | - Citronensäure |
| E 331 | - Natriumcitrat |
| E 332 | - Kaliumcitrat |
| E 333 | - Calciumcitrat |
| E 334 | - Weinsäure |
| E 335 | - Natriumtartrat |
| E 336 | - Kaliumtartrat |
| E 337 | - Natrium-Kaliumtartrat |
| E 338 | - Phosphorsäure |
| E 353 | - Metaweinsäure |
| E 354 | - Calciumtartrat |
| E 355 | - Adipinsäure |
| E 363 | - Bernsteinsäure |
| E 380 | - Triammoniumcitrat |
| E 513 | - Schwefelsäure |
| E 574 | - Gluconsäure |
| E 575 | - Glucono-delta-Lacton |

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:

| | |
|---|---|
| E 170 | - Calciumcarbonat |
| E 260-263 | - Essigsäure und Acetate |
| E 270 | - Milchsäure |
| E 296 | - Äpfelsäure |
| E 297 | - Fumarsäure |
| E 325-327 | - Lactate (Milchsäure) |
| E 330-333 | - Citronensäure und Citrate |
| E 334-337 | - Weinsäure und Tartrate |
| E 339-341 | - Orthophosphate |
| E 350-352 | - Malate (Äpfelsäure) |
| E 450-452 | - Di-, Tri- und Polyphosphate |
| E 500-504 | - Carbonate (Kohlensäure) |
| E 507 | - Salzsäure und ChlorideE 513-517Schwefelsäure und Sulfate |
| E 524-528 | - Hydroxide |
| E 529-530 | - Oxide |
| E 355-357 | - Adipinsäure und Adipate |
| E 574-578 | - Gluconsäure und Gluconate |

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:

| | |
|---|---|
| E 400 | - Alginsäure |
| E 401 | - Natriumalginat |
| E 402 | - Kaliumalginat |
| E 403 | - Ammoniumalginat |
| E 404 | - Calciumalginat |
| E 405 | - Propylenglycolalginat |
| E 406 | - Agar Agar |
| E 407 | - Carrgeen, Furcelleran |
| E 407 | - Johannisbrotkernmehl |
| E 412 | - Guarkernmehl |
| E 413 | - Traganth |
| E 414 | - Gummi arabicum |
| E 415 | - Xanthan |
| E 416 | - Karaya (IndischerTraganth) |
| E 417 | - Tarakernmehl (Peruanisches Johannisbrotkernmehl) |
| E 418 | - Gellan |
| E 440 | - Pektin, Opekta |
| E 440ii | - Amidiertes Pektin |
| E 460 | - Mikrokristalline Cellulose, Cellulosepulver |
| E 461 | - Methylcellulose |
| E 462 | - Ethylcellulose |
| E 463 | - Hydroxypropylcellulose |
| E 465 | - Methylethylcellulose |
| E 466 | - Carboxymethylcellulose, Natriumcarboxymethylcellulose |

### Aromastoffe

Weiterhin können die oben genannten erfindungsgemäßen oralen Zubereitungen und Nahrungsmitteln eine oder mehrere Aromastoffe enthalten. Typische Beispiele umfassen: Acetophenon, Allylcapronat, alpha-lonon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Insbesondere sind die Aromastoffe hervorzuheben, die einen milchig-sahnigen und Mundfülle gebenden Geschmackseindruck vermitteln (wie z.B. Diacetyl, Acetoin oder delta-Lactone) und Aromastoffe, die einen süss-karamelligen Geschmackseindruck vermitteln, der ein Zuckerprofil unterstützt (wie z.B. Maltol, Vanillin, Benzaldehyd, Furaneol, Heliotropin).

### Aromastoffe zur Maskierung von unangenehmen Geschmackseindrücken

Weiterhin können die oben genannten erfindungsgemäßen oralen Zubereitungen und Nahrungsmitteln vor allem zusätzliche Aromastoffe enthalten, die einen unangenehmen (bitteren, adstringierenden, trockenen, mehligen, metallischen, kalkigen, kreidigen) Geschmack oder Nachgeschmack der Zubereitungen verringern oder unterdrücken können, sogenannte Geschmackskorrigenzien. Diese Geschmackskorrigenzien werden z. B. aus der folgenden Liste ausgewählt: Lactisole, Lactisolester wie in EP 2,292,224 beschrieben, Natriumsalze (z.B. Natriumchlorid, Natriumlactat, Natriumcitrat, Natriumacetat, Natriumgluconoat), Hydroxyflavanone, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1258200 A2, die bezüglich der darin offenbarten entsprechenden Verbindungen auf dem Wege der Verweisung Bestandteil dieser Anmeldung wird), Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen (vorzugsweise wie beschrieben in US 2008/0227867 A1, die bezüglich der darin offenbarten entsprechenden Verbindungen auf dem Wege der Verweisung Bestandteil dieser Anmeldung wird), dabei insbesondere Phloretin und Davidigenin, Umami-Verbindungen wie in WO 2008/046895 A1, EP 1989944 A1 oder beschrieben, Umami-Verbindungen wie beschrieben in EP 2064959 A1, EP 2,529,632 -B1 bzw. EP 2135516 A1, dabei besonders bevorzugt Rubemamin und Rubescenamin, Vanillyllignanen, besonders bevorzugt Lariciresinol oder Matairesinol wie in WO 2012 146,584 beschrieben, Enterodiol wie in DE 102 012,214,560 beschrieben, Alkamiden, insbesondere Pellitorinen wie in EP 2,058,297, EP 1,977,655-B1 und EP 2,008,530-B1 beschrieben, sowie N-Decadienoylaminosäuren wie in EP 2,597,082 beschrieben, und deren Gemische.

### MUND- UND ZAHNPFLEGEMITTEL

In einer bevorzugten Ausführungsform können die oralen Zubereitungen ausgewählt sein aus der Gruppe, die gebildet wird von Mund- und Zahnpflegemitteln zu denen auch Mundwässer und Kaugummis gezählt werden.

Konkrete Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan-1.1-diphosphonsäure, 1-Phosphonpropan-1,2,3-tricarbonsäure und andere, die z. B. aus US 3,488,419, DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stevia-extrakte oder deren süßenden Bestandteile, insbesondere Rebaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### KAUGUMMIS

Soweit es sich bei den oralen Zubereitungen um Kaugummis handelt, enthalten diese typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### GEWERBLICHE ANWENDBARKEIT

Weiterer Gegenstand der vorliegenden Erfindung ist die Verwendung der erfindungsgemäßen Stoffmischung zur Herstellung von Aromazubereitungen. Die so hergestellten Aromazubereitungen sind besonders vorteilhaft, insbesondere zur Verwendung zum Verbessern, Verstärken oder Vermitteln des Süßgeschmacks und / oder zum Überdeckung eines unangenehmen Geschmacksimpression, beispielsweise von Süßungsmittel, bevorzugt ausgewählt aus bitter, adstringierend und/oder Süßungsmittel ähnlichem (Nach-) Geschmack.

Demgemäß ist ein weiterer Gegenstand der vorliegenden Erfindung die Verwendung der erfindungsgemäßen Stoffmischungen zum Verbessern, Verstärken oder Vermitteln des Süßgeschmacks und / oder zum Überdeckung eines unangenehmen Geschmacksimpression, beispielsweise von Süßungsmittel, bevorzugt ausgewählt aus bitter, adstringierend und/oder Süßungsmittel ähnlichem (Nach-) Geschmack.

Zudem ist daher ein Gegenstand der Erfindung eine Aromazubereitung, umfassend die oben beschriebenen erfindungsgemäßen Stoffmischungen.

Insbesondere betrifft die Erfindung oral konsumierbare Zubereitung, umfassend eine erfindungsgemäße Stoffmischung oder eine erfindungsgemäße Aromazubereitung, welche die erfindungsgemäßen Stoffmischungen umfassen.

Weiterhin betrifft die Erfindung ein Verfahren zum Verbessern, Verstärken oder Vermitteln des Süßgeschmacks oder zur Überdeckung von unangenehmen Geschmacksimpressionen, ausgewählt aus bitter, adstringierend und/oder Süßungsmittel ähnlichem (Nach-) Geschmack, umfassend die Schritte:
(a) Bereitstellung eines Süßungsmittel, welches einen unangenehmen Geschmackimpression im Mundbereich bewirkt,
(b) Bereitstellung einer Stoffmischung nach einem der Ansprüche 1 bis 10,
(c) Mischen der Komponenten aus Schritt a) und b).

Weitere Gegenstände der vorliegenden Erfindung sind zum einen auf ein Verfahren zur geschmacklichen Optimierung von Zubereitungen für die orale Aufnahme gerichtet, das sich dadurch auszeichnet, dass man diesen mit 0,0001 Gew.-% bis 2 Gew.-% der Mischungen, umfassend die Komponenten
(a) Rubusglykoside und / oder alpha-Glycosylrubusoside, und
(b) Stärkeabbauprodukte, und
(c) optional, ein oder mehrere phenolische, natürlich vorkommende süßverstärkende Aromastoffe ausgewählt aus der Gruppe, die gebildet wird von Hesperetin, Phloretin, 1-(2,4-Dihydroxy-phenyl)-3-(3-hydroxy-4-methoxy-phenyl)-propan-1-on, 7,3-Dihydroxy-4'methoxyflavan und 5-Hy¬droxy-4-(4-hydroxy-3-methoxy-phenyl)-7-methoxy-2-chromanon,
zusetzt.

Schließlich betrifft die Erfindung die Verwendung der Stoffmischungen enthaltend die Komponenten
(a) Rubusglykoside und / oder alpha-Glycosylrubusoside, und
(b) Stärkeabbauprodukte, und
(c) optional, ein oder mehrere phenolische, natürlich vorkommende süßverstärkende Aromastoffe ausgewählt aus der Gruppe, die gebildet wird von Hesperetin, Phloretin, 1-(2,4-Dihydroxy-phenyl)-3-(3-hydroxy-4-methoxy-phenyl)-propan-1-on, 7,3-Dihydroxy-4'methoxyflavan und 5-Hy¬droxy-4-(4-hydroxy-3-methoxy-phenyl)-7-methoxy-2-chromanon,
als Aromazubereitungen für Zubereitungen für die orale Aufnahme.

Bezüglich der bevorzugten Zusammensetzungen der Mischungen, den bevorzugten Einsatz- bzw. Zusatzmengen sowie der Natur der Aromastoffe wird auf die vorstehenden Ausführungen verwiesen.

Ein weiterer Gegenstand der vorliegenden Erfindung ist ein Verfahren zum Verbessern, Verstärken oder Vermitteln des Süßgeschmacks oder zur Überdeckung von unangenehmen Geschmacksimpressionen, ausgewählt aus bitter, adstringierend und/oder Süßungsmittel ähnlichem (Nach-) Geschmack, umfassend die Schritte:
(a) Bereitstellung eines Süßungsmittel, welches einen unangenehmen Geschmackimpression im Mundbereich bewirkt,
(b) Bereitstellung einer Stoffmischung nach einem der Ansprüche 1 bis 10,
(c) Mischen der Komponenten aus Schritt a) und b).

### BEISPIELE

### BEISPIEL 1

### Herstellungsbeispiel von alpha-Glycosylrubusosiden

Maltodextrin DE 17-20 aus Kartoffel und Brombeerblätterextrakt enthaltend 70% Rubusosid werden im Verhältnis 1:1 eingewogen. Danach wird unter Zugabe von 0,1 M Natriumphosphatpuffer, pH 7 gerührt bis alle Komponenten vollständig gelöst sind. Die Reaktion wird durch Zugabe von 1,4 KNU-CP Cyclomaltodextrin glucanotransferase aus pro Gramm Substrat gestartet. Das Gemisch wird nachfolgend für 15 h bei 50 °C unter leichtem Schütteln inkubiert, für 10 min auf 80°C erhitzt und anschließend gefriergetrocknet. Die Analyse des Gemisches erfolgte mittels LC-MS und AF4.

**Tabelle 1**

| Zusammensetzung von Stoffgemischen für Aromazubereitungen, enthaltend alpha-Glucosylrubusoside und Stärkeabbauprodukte (Mengenangabe in Flächen-%) | | | | | | | | | | | | |
|---|---|---|---|---|---|---|---|---|---|---|---|---|
| **Inhaltsstoffe** | **A** | **B** | **C** | **D** | **E** | **F** | **G** | **H** | **I** | **J** | **K** | **L** |
| Steviolmonoside | 2,3 | 2,1 | 1,4 | 1,9 | 1,8 | 1,9 | 1,4 | 5,6 | 1,6 | 2,4 | 2,8 | 1,6 |
| Rubusosid | 15,1 | 20,4 | 13,9 | 16,2 | 16,9 | 19,2 | 15,7 | 20,1 | 18,1 | 23,2 | 28,3 | 17,9 |
| Rubustriglucoside | 13,5 | 18,0 | 14,6 | 15,9 | 15,5 | 17,1 | 15,8 | 17,7 | 17,2 | 18,0 | 21,7 | 16,7 |
| Rubustetraglucoside | 20,4 | 23,6 | 18,2 | 18,8 | 18,5 | 19,1 | 14,9 | 19,5 | 22,2 | 21,1 | 21,9 | 18,1 |
| Rubuspentaglucoside | 13,1 | 11,6 | 18,9 | 17,6 | 17,8 | 15,9 | 17,9 | 15,7 | 16,3 | 15,3 | 10,0 | 16,0 |
| Rubushexaglucoside | 9,3 | 5,1 | 10,3 | 9,6 | 9,7 | 8,5 | 9,8 | 8,2 | 7,8 | 7,0 | 3,0 | 8,8 |
| Rubusheptaglucoside | 2,8 | 1,9 | 5,2 | 4,8 | 4,9 | 4,0 | 4,9 | 3,6 | 3,6 | 3,2 | 0,9 | 3,7 |
| Stärkeabbauprodukte | 4,5 | 6,1 | 4,9 | 6,4 | 6,1 | 6,6 | 1,9 | 5,5 | 4,2 | 3,3 | 4,6 | 7,6 |
| **Summe** | 81,0 | 88,8 | 87,4 | 91,2 | 91,2 | 92,3 | 82,3 | 95,9 | 91 | 93,5 | 93,2 | 90,4 |

### BEISPIEL 2

### Aromazubereitungen

Die unten stehenden Komponenten werden für die entsprechenden Aromazubereitungen zusammengemischt.

**Tabelle 2**

| Aromazubereitungen (Mengenangabe als Gew.-%) | | | | | | | | | | |
|---|---|---|---|---|---|---|---|---|---|---|
| **Zusammensetzung** | **A** | **B** | **C** | **D** | **E** | **F** | **G** | **H** | **I** | **J** |
| Stoffgemisch A enthaltend Maltodextrine aus Beispiel 1 | 10 | 20 | 10 | | | 10 | | 10 | | |
| Stoffgemisch E enthaltend Maltodextrine aus Beispiel 1 | | | | 20 | 15 | | 7,5 | | | |
| Stoffgemisch F Beispiel 1 | | | | | | | | | 20 | |
| Stoffgemisch I Beispiel 1 | | | | | | | | | | 20 |
| Glycerin | 10 | 20 | | 30 | | 20 | 15 | 30 | 10 | 20 |
| Gummi arabicum-Lösung (20%) | | | | | | | | 10 | | |
| 1,2-Propylenglycol | Ad 100 | | | | | | | | | |

### BEISPIEL 3

### Getränkeformulierung

Verschiedene erfindungsgemäße Stoffgemische wurden zur Herstellung von einfachen Softdrinks verwendet und die Produkte 48 h bei 20°C gelagert. Anschließend wurden die geschmacklichen Eigenschaften (Deskriptoren: Anfangssüße, Süßintensität, Zuckergeschmack/Mundfülle) von einem Panel bestehend aus 8 geschulten Testern auf einer Linien-Skala von 0 (nicht vorhanden) bis 10 (stark ausgeprägt) bewertet. Die Zusammensetzungen und Ergebnisse sind in der nachfolgenden **Tabelle 3** zusammengefasst. Die Ausführungen 1 und 2 sind erfindungsgemäß, die Beispiele V1, V2 und V3 dienen zum Vergleich. Das Beispiel C entspricht dem Standard, d.h. der geschmacklichen Beurteilung des Produktes ohne Zusatz von Aroma.

**Tabelle 3**

| Geschmackliche Eigenschaften von Softdrinkformulierungen (Mengenangabe als Gew.-%) | | | | | | |
|---|---|---|---|---|---|---|
| **Zusammensetzung** | **C** | **1** | **2** | **V1** | **V2** | **V3** |
| Sucrose | 7 | 7 | 7 | 7 | 7 | 7 |
| Zitronensäure | 0.15 | 0.15 | 0.15 | 0.15 | 0.15 | 0.15 |
| Stoffgemisch A enthaltend Maltodextrine aus Beispiel 1 | - | 0.01 | 0.01 | - | - | - |
| Hesperetin | - | | 0.00075 | 0.00075 | - | 0,00075 |
| Brombeerblätterextrakt enthaltend 70 % Rubusosid | - | - | - | - | 0,005 | 0,005 |
| Maltodextrin | - | - | - | - | 0,005 | 0,005 |
| Wasser | Ad 100 | | | | | |
| Anfangssüße | 3,4 | 5,5 | 5,6 | 3,5 | 4,5 | 5,0 |
| Süßintensität | 3,6 | 5,9 | 6,4 | 3,9 | 4,9 | 5,4 |
| Zuckergeschmack und Mundfülle | 3,3 | 5,1 | 5,9 | 3,6 | 4,5 | 5,1 |
| Anhaltender Süßgeschmack, Süßstoffartigkeit | 2,1 | 3,1 | 2,8 | 2,1 | 2,7 | 3,3 |

Die zuckerreduzierte Kontrollprobe C wird eindeutig am schlechtesten bewertet, die Probe 2 dagegen am besten. Die Zugabe von geringen Mengen Hesperetin (V1) verbessert dabei die Geschmackswerte nicht. Die beiden erfindungsgemäßen Ausführungen 1 und 2 zeigen eine verbesserte Anfangssüße, erhöhte Süßintensität und einen verbesserten Zuckergeschmack/Mundfülle gegen über der Zuckervariante (C). Dabei zeigt die Ausführung 1 im Vergleich mit der Probe V2 eine höhere Süßintensität und Anfangssüße sowie eine verbesserte Mundfülle. Die Kombination von alpha-Glycosylrubussosiden mit (2) führt zum einen zu einer weiteren Verstärkung der Süßintensität und der Mundfülle gegenüber der Variante mit alpha-Glycosylrubussosiden allein (1) sowie der Variante mit Rubusosid und Hesperetin (V3). Zum Anderen führt die Kombination von Hesperetin mit alpha-Glycosylrubussosiden in der erfindungsgemäßen Ausführung (2) zu einer Maskierung der Süßstoffartigkeit bzw. des Lakritz-artigen Nachgeschmacks im Vergleich zur Kombination von Rubusosid mit Hesperertin (V3)

### BEISPIELE 4 bis 9:

Im Folgenden wird die Erfindung an Hand weiterer Formulierungsbeispiele illustriert; alle Mengenangaben als Gew.-%.

### Beispiel 4: Erfrischungsgetränke

| **Inhaltsstoffe** | **A** | **B** | **C** | **D** | **E** | **F** | **G** |
|---|---|---|---|---|---|---|---|
| Sucrose | 10 | 10 | 7 | - | - | 8 | 7 |
| Glucose/Fructose Sirup | - | - | - | - | 10 | - | - |
| Stoffgemisch B enthaltend Maltodextrine aus Beispiel 1 | 0,01 | 0,015 | 0,005 | 0,02 | 0,01 | 0,005 | 0,002 |
| Zitronensäure | 0,15 | 0,15 | 0,06 | 0,15 | 0,15 | 0,15 | 0,15 |
| Phosphorsäure | - | - | 0,07 | - | - | - | - |
| Zuckercoleur | - | - | 0,14 | - | - | - | - |
| Koffein | - | - | 0,01 | - | - | - | - |
| Zitrusaroma | 0,1 | 0,05 | - | 0,1 | 0,1 | 0,1 | 0,1 |
| Limonenaroma | - | 0,05 | - | - | - | - | - |
| Getränkeemulsion Typ "Cola" | - | - | 0,05 | - | - | - | - |
| Phloretin | - | - | - | 0,0005 | - | 0,0005 | 0,01 |
| Hesperetin | 0,00075 | | 0,00075 | 0,0012 | | 0,00075 | 0,01 |
| Homoeriodictyol-Na | - | - | 0,005 | 0,005 | - | - | - |
| Wasser | Ad 100 | | | | | | |

Die Zutaten wurden in der angegebenen Reihenfolge gemischt und mit Wasser auf 100 % aufgefüllt. Die Mischungen werden in Glasflaschen gefüllt und carbonisiert.

### Beispiel 5: Hartkaramellen

| **Inhaltsstoffe** | **A** | **B** | **C** | **D** |
|---|---|---|---|---|
| Zucker | 74,50 | - | - | - |
| Palatinit, Type M | - | 74,00 | 75,50 | 75,00 |
| Zitronensäure | 0,5 | 1,0 | 0,5 | - |
| Farbstoff, gelb | - | 0,01 | - | - |
| Farbstoff, rot | - | - | 0,01 | - |
| Farbstoff, blau | 0,01 | - | - | 0,01 |
| Pfefferminzaroma | 0,1 | - | - | 0,1 |
| Zitrusaroma | - | 0,1 | - | - |
| Rote Beeren-Aroma | - | - | 0,1 | - |
| Stoffgemisch C enthaltend Maltodextrine aus Beispiel 1 | 0,01 | 0,012 | 0,015 | 0,008 |
| Balansin A | - | 0,005 | 0,010 | 0,005 |
| Hesperetin | | 0,001 | 0,0005 | |
| Phloretin | - | 0,002 | - | - |
| Wasser | ad 100 | ad 100 | ad 100 | ad 100 |

| | | | | |
|---|---|---|---|---|
| ¹⁾ Steviolglykosid/Maltodextrin-Mischung (80:20) der Firma Pure Circle | | | | |

### Beispiel 6: Joghurt mit niedrigem Fettgehalt

| **Inhaltsstoffe** | **A** | **B** | **C** | **D** |
|---|---|---|---|---|
| Sucrose | 10 | 8 | 6 | - |
| Sucralose | - | 0,02 | - | 0,2 |
| Rebaudiosid A > 95 % | - | - | 0,025 | - |
| Saccharin | | - | - | 0,3 |
| Stoffgemisch D enthaltend Maltodextrine aus Beispiel 1 | 0,01 | 0,012 | 0,02 | 0,1 |
| Sauerkirsch-Extrakt nach Beispiel 1 | 0,2 | 0,1 | 0,2 | 0,2 |
| Hesperetin | | 0,001 | | 0,002 |
| Phloretin | - | - | 0,002 | |
| Homoeriodictyol-Natriumsalz | - | - | - | 0,005 |
| Joghurt, 0,1 % Fett | zu 100 % auffüllen | | | |

### Beispiel 7: Fruchtgummis

| **Inhaltsstoffe** | **A** | **B** | **C** |
|---|---|---|---|
| Saccharose | 34,50 | 8,20 | 34,50 |
| Glucosesirup, DE 40 | 31,89 | 30,09 | 31,89 |
| Stoffgemisch E enthaltend Maltodextrine aus Beispiel 1 | 0,01 | 0,012 | 0,01 |
| Iso Sirup C* Tru Sweet 01750 (Cerestar GmbH) | 1,50 | 2,10 | 1,50 |
| Gelatine 240 Bloom | 8,20 | 9,40 | 8,20 |
| Polydextrose (Litesse® Ultra, Danisco Cultor GmbH) | - | 24,40 | - |
| Farbstoff | 0,01 | 0,01 | 0,01 |
| Zitrusaroma | 0,20 | - | 0,20 |
| Kirscharoma | - | 0,10 | - |
| Hesperetin | 0,0075 | | - |
| Wasser | ad 100 | ad 100 | ad 100 |

### Beispiel 8: Zuckerfreies Kaugummi

| **Inhaltsstoffe** | **Gehalt** |
|---|---|
| Kaugummibasis | 30.00 |
| Sorbitolpulver | Ad 100 |
| Stoffgemisch F enthaltend Maltodextrine aus Beispiel 1 | 0,1 |
| Palatinit | 9.50 |
| Xylitol | 2.00 |
| Mannitol | 3.00 |
| Aspartam | 0.10 |
| Acesulfam K | 0.10 |
| Emulgum / Emulgator | 0.30 |
| Sorbitol 70 %. in Wasser | 14.00 |
| Glycerin | 1.00 |
| Pfefferminzaroma | 1.50 |
| Hesperetin | 0,01 |

### Beispiel 9: Pudding

| **Inhaltsstoffe (g)** | **A** | **B** | **C** |
|---|---|---|---|
| Maisstärke | 38 | 38 | 38 |
| Zucker | 38 | 30 | 22,8 |
| Stoffgemisch G enthaltend Maltodextrine aus Beispiel 1 | - | 0,1 | 0,15 |
| Hesperetin | - | | 0,03 |
| Vanille-Aroma (Symrise) | 0,2 | 0,2 | 0,2 |
| Chinolin gelb | 0,02 | 0,02 | 0,02 |
| Milch | 500 | 500 | 500 |

## Patentansprüche

1. Stoffmischung, enthaltend mindestens eine Verbindung der allgemeinen Formel (I), 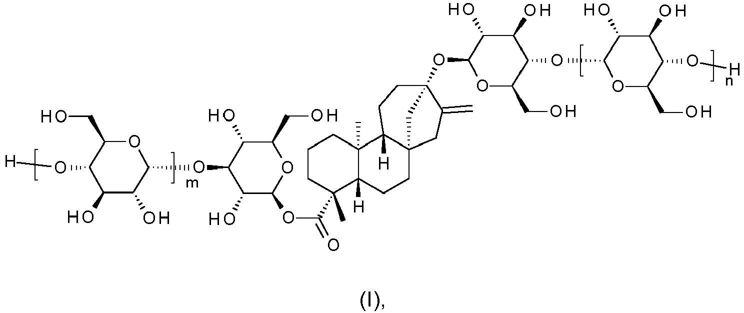 wobei
m und n unabhängig voneinander 0 bis 50 darstellen,
und wobei im Falle von m = n = 0 mindestens eine weitere Verbindung der allgemeinen Formel (I) mit m oder n ≥ 1 anwesend ist,
**dadurch gekennzeichnet, dass** sie
(a1) weniger als 50 Gew.-% Rubusosid (m + n = 0),
(a2) mehr als 10 Gew.-% alpha-Glucosylrubusoside der oben angegebenen allgemeinen Formel (I) mit insgesamt 3 Glucoseeinheiten (m + n = 1),
(a3) mehr als 15 Gew.-% alpha-Glucosylrubusoside der allgemeinen Formel (I) mit insgesamt 4 Glucoseeinheiten (m + n = 2),
(a4) mehr als 5 Gew.-% alpha-Glucosylrubusoside der allgemeinen Formel (I) mit insgesamt 5 Glucoseeinheiten (m + n = 3), und
(a5) weniger als 6 Gew.-% Steviolmonosid,
aufweist,
wobei die Menge aller Komponenten (a1) bis (a5) sich zusammen zu mindestens 80 Gew.-% addiert und die restliche Menge sich aus Wasser, Glycerin, Stärkeabbauprodukten, Proteinen, Fettsäuren und/ oder Fettsäureestern zusammensetzt.

2. Stoffmischung nach Anspruch 1, **dadurch gekennzeichnet, dass** sie
(a1) weniger als 30 Gew.-% Rubusosid (m + n = 0),
(a2) mehr als 15 Gew.-% alpha-Glucosylrubusoside mit insgesamt 3 Glucoseeinheiten (m + n = 1),
(a3) mehr als 18 Gew.-% alpha-Glucosylrubusoside mit insgesamt 4 Glucoseeinheiten (m + n = 2),
(a4) mehr als 10 Gew.-% alpha-Glucosylrubusoside mit insgesamt 5 Glucoseeinheiten (m + n = 3),
(a5) weniger als 2,5 Gew.-% Steviolmonosid,
aufweist, wobei die Menge aller Komponenten (a1) bis (a5) sich zusammen zu mindestens 80 Gew.-% addiert und die restliche Menge sich aus Wasser, Glycerin, Stärkeabbauprodukten, Proteinen, Fettsäuren und/ oder Fettsäureestern zusammensetzt.

3. Stoffmischung nach einem der Ansprüche 1 oder 2, **dadurch gekennzeichnet, dass** sie als Verbindung der Formel (I) alpha-Glycosylruboside der Formel (II) enthält, 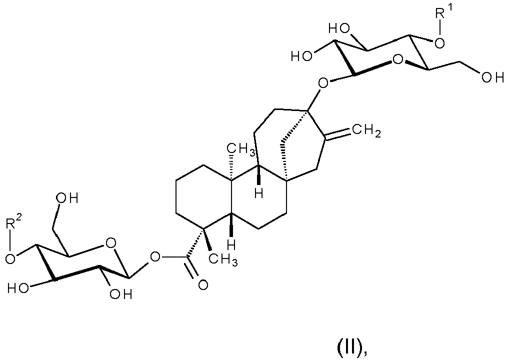 wobei die Reste R¹ und R² die folgenden Bedeutungen besitzen:
| **Verb.** | **Rubusglucosid** | **R¹** | **R²** |
|---|---|---|---|
| 1 | Rubustriglucosid | H- | Glcα1- |
| 2 | Rubustriglucosid | Glcα1- | H- |
| 3 | Rubustetraglucosid | Glcα1- | Glcα1- |
| 4 | Rubustetraglucosid | -Glcα1-4Glcα | H- |
| 5 | Rubustetraglucosid | H- | -Glcα1-4Glcα |
| 6 | Rubuspentaglucosid | -Glcα1-4Glcα | Glcα1- |
| 7 | Rubuspentaglucosid | Glcα1- | -Glcα1-4Glcα |
| 8 | Rubushexaglucosid | -Glcα1-4Glcα | -Glcα1-4Glcα |
| 9 | Rubuspentaglucosid | -Glcα1-4Glcα1-4Glcα | H- |
| 10 | Rubuspentaglucosid | H- | -Glcα1-4Glcα1-4Glcα |
| 11 | Rubushexaglucosid | -Glcα1-4Glcα1-4Glcα | Glcα1- |
| 12 | Rubushexaglucosid | Glcα1- | -Glcα1-4Glcα1-4Glcα |
| 13 | Rubusheptaglucosid | -Glcα1-4Glca | -Glcα1-4Glcα1-4Glcα |
| 14 | Rubusheptaglucosid | -Glcα1-4Glcα1-4Glcα | -Glcα1-4Glcα |
| 15 | Rubusoctaglucosid | -Glcα1-4Glcα1-4Glcα | -Glcα1-4Glcα1-4Glcα |

4. Mischung nach einem der Ansprüche 1 bis 3, **dadurch gekennzeichnet, dass** die Stärkeabbauprodukte 3 bis 100 jeweils alpha-1-4-verknüpften D-Glucopyranosylresten enthalten.

5. Mischung nach einem der Ansprüche 1 bis 4, **dadurch gekennzeichnet, dass** das Gemisch zusätzlich ein oder mehrere phenolische, natürlich vorkommende süßverstärkende Aromastoffe umfasst, ausgewählt aus der Gruppe, die gebildet wird von Hesperetin, Phloretin, 1-(2,4-Dihydroxy-phenyl)-3-(3-hydroxy-4-methoxy-phenyl)-propan-1-on und 5-Hydroxy-4-(4-hydroxy-3-methoxy-phenyl)-7-methoxy-2-chromanon.

6. Verwendung einer Mischung nach einem der Ansprüche 1 bis 5 zur Herstellung von Aromazubereitungen.

7. Verwendung einer Mischung nach einem der Ansprüche 1 bis 5 zum Verbessern, Verstärken oder Vermitteln des Süßgeschmacks und / oder zum Überdeckung eines unangenehmen Geschmacksimpression, beispielsweise von Süßungsmittel, bevorzugt ausgewählt aus bitter, astringent und/oder Süßungsmittel ähnlichem (Nach-) Geschmack.

8. Aromazubereitung, umfassend eine Mischung nach einem der Ansprüchen 1 bis 5.

9. Oral konsumierbare Zubereitung umfassend eine Mischung nach einem der Ansprüche 1 bis 5 oder eine Aromazubereitung nach Anspruch 8.

10. Verfahren zur Herstellung von Rubusglykosiden in einer Stoffmischung nach einem der Ansprüche 1 bis 5, umfassend die Verwendung von alpha-Cyclodextrin glucanotransferase in der Transglucosylierungs-Reaktion mit einem 1,4-glykosidischen Kohlenhydrat, welches vorzugsweise Kohlenhydrat ist.

## Claims

1. Substance mixture comprising at least one compound of the general formula (I), 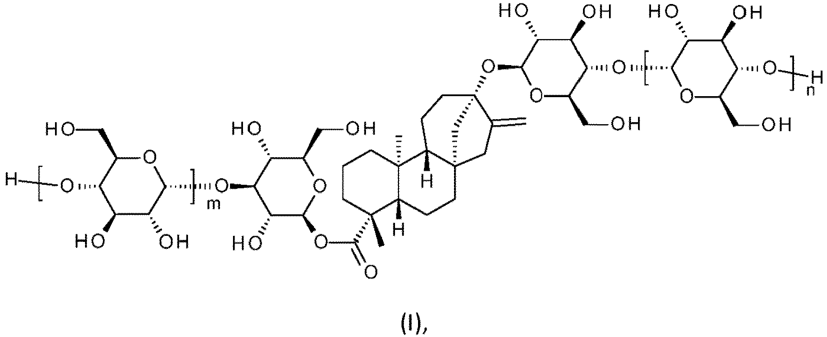 wherein
m and n represent independent of each other 0 to 50,
and wherein in case of m = n = 0 at least one further component of the general formula (I) with m or n ≥ 1 is present,
**characterized in that** it has
(a1) less than 50 wt.-% rubusoside (m + n = 0),
(a2) more than 10 wt.-% alpha-glucosylrubusosides of the general formula (I) depicted above with in total 3 glucose units (m + n = 1),
(a3) more than 15 wt.-% alpha-glucosylrubusosides of the general formula (I) with in total 4 glucose units (m + n = 2),
(a4) more than 5 wt.-% alpha-glucosylrubusosides of the general formula (I) with in total 5 glucose units (m + n = 3), and
(a5) less than 6 wt.-% steviolmonoside,
wherein the amount of all components (a1) to (a5) together add to at least 80 wt.-% and the remaining amount is composed of water, glycerol, starch degradation products, proteins, fatty acids and/or fatty acid esters.

2. Substance mixture according to claim 1, **characterized in that** it has
(a1) less than 30 wt.-% rubusoside (m + n = 0),
(a2) more than 15 wt.-% alpha-glucosylrubusosides with in total 3 glucose units (m + n = 1),
(a3) more than 18 wt.-% alpha-glucosylrubusosides with in total 4 glucose units (m+n=2),
(a4) more than 10 wt.-% alpha-glucosylrubusosides with in total 5 glucose units (m + n = 3), and
(a5) less than 2.5 wt.-% steviolmonoside,
wherein the amount of all components (a1) to (a5) together add to at least 80 wt.-% and the remaining amount is composed of water, glycerol, starch degradation products, proteins, fatty acids and/or fatty acid esters.

3. Substance mixture according to one of the claims 1 or 2, **characterized in that** it comprises alpha-glycosylrubusoides of formula (II) as compound of formula (I), 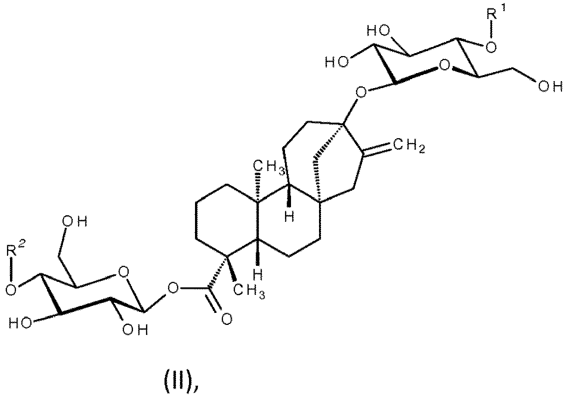 wherein the rests R¹ and R² have the following meaning:
| **Compound** | **Rubusglucoside** | **R¹** | **R²** |
|---|---|---|---|
| 1 | Rubustriglucoside | H- | Glcα1- |
| 2 | Rubustriglucoside | Glcα1- | H- |
| 3 | Rubustetraglucoside | Glcα1- | Glcα1- |
| 4 | Rubustetraglucoside | -Glcα1-4Glcα | H- |
| 5 | Rubustetraglucoside | H- | -Glcα1-4Glcα |
| 6 | Rubuspentaglucoside | -Glcα1-4Glcα | Glcα1- |
| 7 | Rubuspentaglucoside | Glcα1- | -Glcα1-4Glcα |
| 8 | Rubushexaglucoside | -Glcα1-4Glcα | -Glcα1-4Glcα |
| 9 | Rubuspentaglucoside | -Glcα1-4Glcα1-4Glcα | H- |
| 10 | Rubuspentaglucoside | H- | -Glcα1-4Glcα1-4Glcα |
| 11 | Rubushexaglucoside | -Glcα1-4Glcα1-4Glcα | Glcα1- |
| 12 | Rubushexaglucoside | Glcα1- | -Glcα1-4Glcα1-4Glcα |
| 13 | Rubusheptaglucoside | -Glcα1-4Glcα | -Glcα1-4Glcα1-4Glcα |
| 14 | Rubusheptaglucoside | -Glcα1-4Glcα1-4Glcα | -Glcα1-4Glcα |
| 15 | Rubusoctaglucoside | -Glcα1-4Glcα1-4Glcα | -Glcα1-4Glcα1-4Glcα |

4. Mixture according to one of the claims 1 to 3, **characterized in that** the starch degradation products comprise 3 to 100 D-glucopyranosylrests, each being alpha-1-4-linked.

5. Mixture according to one of the claims 1 to 4, **characterized in that** the mixture additionally comprises one or more phenolic, naturally occurring sweetening flavouring agents selected from the group consisting of Hesperetin, Phloretin, 1-(2,4-dihydroxy-phenyl)-3-(3-hydroxy-4-methoxy-phenyl)-propane-1-one and 5-Hydroxy-4-(4-hydroxy-3-methoxy-phenyl)-7-methoxy-2-chromanone.

6. Use of a mixture according to one of the claims 1 to 5 for the production of aroma compositions.

7. Use of a mixture according to one of the claims 1 to 5 to improve, enhance or impart the sweet taste and/or to mask an unpleasant taste impression, for example of sweeteners, preferably selected from bitter, adstringent and/or (after)taste similar to sweeteners.

8. Aroma composition comprising a mixture according to one of the claims 1 to 5.

9. Orally consumable composition comprising a mixture of one of the claims 1 to 5 or an aroma composition according to claim 8.

10. Method for producing rubusglycosides in a substance mixture according to one of the claims 1 to 5 comprising the use of alpha-cyclodextrin glucanotransferase in the transglycosylation reaction with a 1,4-glycosydic carbohydrate, which is preferably carbohydrate.

## Revendications

1. Mélange de substances comprenant au moins un composé de formule générale (I), 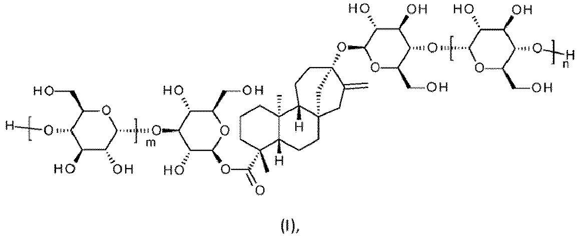 dans lequel
m et n représentent, indépendamment l'un de l'autre, 0 à 50,
et, dans le cas où m = n = 0, au moins un autre composé de formule générale (I), avec m ou n ≥ 1, est présent,
**caractérisé par le fait qu'**il comprend
(a1) moins de 50 % en poids de rubusoside (m + n = 0),
(a2) plus de 10 % en poids d'alpha-glycosylrubusoside de formule générale (I) mentionnée ci-dessus, ayant dans l'ensemble 3 unités de glucose (m + n = 1),
(a3) plus de 15 % en poids d'alpha-glycosylrubusoside de formule générale (I) ayant dans l'ensemble 4 unités de glucose (m + n = 2),
(a4) plus de 5 % en poids d'alpha-glycosylrubusoside de formule générale (I) ayant dans l'ensemble 5 unités de glucose (m + n = 3), et
(a5) moins de 6 % en poids de monoside de stéviol,
dans lequel la quantité de l'ensemble des composants (a1) à (a5) s'additionne pour donner ensemble au moins 80 % en poids, et la quantité restante se compose d'eau, de glycérol, de produits de décomposition d'amidon, de protéines, d'acides gras et/ou d'esters d'acides gras.

2. Mélange de substances selon la revendication 1, **caractérisé par le fait qu'**il comprend
(a1) moins de 30 % en poids de rubusoside (m + n = 0),
(a2) plus de 15 % en poids d'alpha-glycosylrubusoside ayant dans l'ensemble 3 unités de glucose (m + n = 1),
(a3) plus de 18 % en poids d'alpha-glycosylrubusoside ayant dans l'ensemble 4 unités de glucose (m + n = 2),
(a4) plus de 10 % en poids d'alpha-glycosylrubusoside ayant dans l'ensemble 5 unités de glucose (m + n = 3),
(a5) moins de 2,5 % en poids de monoside de stéviol,
dans lequel la quantité de l'ensemble des composants (a1) à (a5) s'additionne pour donner ensemble au moins 80 % en poids, et la quantité restante se compose d'eau, de glycérol, de produits de décomposition d'amidon, de protéines, d'acides gras et/ou d'esters d'acides gras.

3. Mélange de substances selon l'une quelconque des revendications 1 ou 2, **caractérisé par le fait qu'**il contient, en tant que composé de formule (1), des alpha-glycosylrubusosides de formule (II), 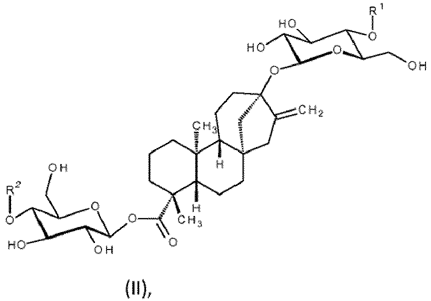 dans lequel les restes R¹ et R² présentent les significations suivantes:
| composé | rubusglucoside | R¹ | R² |
|---|---|---|---|
| 1 | rubustriglucoside | H- | Glcα1- |
| 2 | rubustriglucoside | Glcα1- | H- |
| 3 | rubustétraglucoside | Glcα1- | Glcα1- |
| 4 | rubustétraglucoside | -Glcα1-4Glcα | H- |
| 5 | rubustétraglucoside | H- | -Glcα1-4Glcα |
| 6 | rubuspentaglucoside | -Glcα1-4Glcα | Glcα1- |
| 7 | rubuspentaglucoside | Glcα1- | -Glcα1-4Glcα |
| 8 | rubushexaglucosid | -Glcα1-4Glcα | -Glcα1-4Glcα |
| 9 | rubuspentaglucoside | -Glcα1-4Glcα1-4Glcα | H- |
| 10 | rubuspentaglucoside | H- | -Glcα1-4Glcα1-4Glcα |
| 11 | rubushexaglucoside | -Glcα1-4Glcα1-4Glcα | Glcα1- |
| 12 | rubushexaglucoside | Glcα1- | -Glcα1-4Glcα1-4Glcα |
| 13 | rubusheptaglucosid | -Glcα1-4Glcα | -Glcα1-4Glcα1-4Glcα |
| 14 | rubusheptaglucoside | -Glcα1-4Glcα1-4Glcα | -Glcα1-4Glcα |
| 15 | rubusoctaglucoside | -Glcα1-4Glcα1-4Glcα | -Glcα1-4Glcα1-4Glcα |

4. Mélange selon l'une quelconque des revendications 1 à 3, **caractérisé par le fait que** les produits de décomposition d'amidon 3 à 100 contiennent chacun des restes de D-glucopyranosyle à liaison alpha-1-4.

5. Mélange selon l'une quelconque des revendications 1 à 4, **caractérisé par le fait que** le mélange comprend en outre un ou plusieurs substances aromatisantes phénoliques, d'origine naturelle et intensifiant le goût sucré qui sont choisis dans le groupe constitué par l'hespérétine, la phlorétine, le 1-(2,4-dihydroxy-phényl)-3-(3-hydroxy-4-méthoxy-phényl)-propan-1-one et le 5-hydroxy-4-(4-hydroxy-3-méthoxy-phényl)-7-méthoxy-2-chromanone.

6. Utilisation d'un mélange selon l'une quelconque des revendications 1 à 5 pour la préparation de compositions aromatisantes.

7. Utilisation d'un mélange selon l'une quelconque des revendications 1 à 5, pour améliorer, renforcer ou conférer le goût sucré et/ou pour masquer une impression de goût désagréable, par exemple d'édulcorant, de préférence choisi parmi amer, astringent et/ou le (arrière-)goût semblable à celui d'un édulcorant.

8. Composition aromatisante comprenant un mélange selon l'une quelconque des revendications 1 à 5.

9. Composition consommable par voie orale, comprenant un mélange selon l'une quelconque des revendications 1 à 5 ou une composition aromatisante selon la revendication 8.

10. Procédé de préparation de rubusglycosides dans un mélange de substances selon l'une quelconque des revendications 1 à 5, comprenant l'utilisation d'alpha-cyclodextrine glucanotransférase dans la réaction de transglycosylation avec un hydrate de carbone 1,4-glycosidique qui est de préférence de l'hydrate de carbone.
